# EUROPEAN PATENT SPECIFICATION

(11) **EP 4 355 951 B1**
(45) Date of publication and mention of the grant of the patent: **05.03.2025**
(21) Application number: 22737997.1
(22) Date of filing: 13.06.2022
(51) Int. Cl.: D21H 17/28, B65D 65/40, B65D 65/42, D21H 21/16, D21H 21/20, D21H 27/10, A61B 50/00

(54) **FIBROUS STERILIZABLE MATERIAL FOR PACKAGING OF MEDICAL DEVICES AND TRAY OBTAINED FROM THE MATERIAL**
FASERIGES STERILISIERBARES MATERIAL ZUR VERPACKUNG MEDIZINISCHER VORRICHTUNGEN UND AUS DIESEM MATERIAL HERGESTELLTE SCHALE
MATÉRIAU FIBREUX STÉRILISABLE POUR L'EMBALLAGE DE DISPOSITIFS MÉDICAUX ET PLATEAU OBTENU À PARTIR DE CE MATÉRIAU

(30) Priority: 14.06.2021 FR 2106237
(43) Date of publication of application: 24.04.2024
(73) Proprietor: Ahlstrom Oyj, 00100 Helsinki (FI)
(72) Inventor: DUFOUR, Menno, 69007 Lyon (FR); LALOUM, Jonathan, 75010 Paris (FR)
(74) Representative: Hoffmann Eitle
(86) International application number: PCT/IB2022/055470
(87) International publication number: WO 2022/264009

(56) References cited:
- WO-A1-99/00244
- WO-A1-99/00549

## Description

### FIELD OF THE DISCLOSURE

The present disclosure relates to the field of sterilizable packages, preferably heat-sealable ones, for medical devices, in particular reusable medical devices intended to be sterilized.

The packages of the present disclosure are intended to be sterilized by suitable means, in particular and without limitation, steam, ethylene oxide, formaldehyde and/or gamma rays.

### BACKGROUND OF THE DISCLOSURE

Two types of sterilizable packaging are available for the most part, especially at hospitals.

First of all, there are boxes or rigid containers made of metal. Due to their metallic nature, they can be particularly robust. However, these boxes have the drawback of their weight and they need to be washed regularly prior to use. Thus, this leads to a substantial handling of them, not to mention that the box eventually becomes worn out and develops leaks. The containers then need to be repaired, which leads to not insignificant costs.

There has been an effort to replace these boxes with disposable flexible packages of the pouch or sheet type. It is clear that these packages are easier to use on account of their weight and their disposable nature. However, they can have inadequate mechanical strength in regard to certain medical devices being sterilized. Furthermore, they are often manufactured from non-renewable resources, which is especially the case with the majority of those manufactured from polypropylene.

Document WO 2017/168152 A1 describes a portable and disposable tray for surgical instruments. The tray is obtained by thermoforming of pulp. In practice, the instrument is stored in a sterilization bag and then positioned within the tray prior to the actual sterilization procedure. The properties of the tray make it permeable to the means of sterilization in the same way as the sterilization bag. The drawback of this system is thus the need for two elements, namely, a tray and a bag, which significantly increases the cost.

Document EP 2917408 A1 describes a cellulose-based paper with a weight between 40 and 120 g/m². The paper is reinforced on one of its surfaces and has a sealing layer on the other surface, allowing the package to be closed by means of a film of polypropylene for example, after inserting the instrument to be sterilized. Thus, in this case there is no intermediate bag. The proposed paper has the major shortcoming of being excessively pliant, making it vulnerable when the instruments being sterilized have sharp edges.

Document GB 2449418A1 describes a sheet of calendered spunbond type, composed of fibers of polypropylene, thus not being biosourced and not being biodegradable.

Documents WO99/00244A1 and WO99/00549A1 disclose a sterilizable paper package for medical applications, comprising cellulosic fibers. These documents are silent about the maximum size of the pores of said fibrous structures.

### SUMMARY OF THE DISCLOSURE

To the knowledge of the Applicant, no disposable packaging rigid enough to make into trays, for example, has yet been proposed in hospitals for the sterilization of medical equipment.

Consequently, a problem which the present disclosure proposes to solve is to provide a rigid disposable packaging which is resistant to tearing, in particular, and which is ideally obtained from renewable and/or compostable components.

More precisely, an objective of the present disclosure is to provide a rigid disposable material which enables the sterilizing of instruments which are intended to be sterilized without having a risk of infection with microorganisms. Thus, it needs to be both permeable to the sterilization agent, which is in practice, for example, steam, ethylene oxide, formaldehyde, and gamma rays, and a barrier to bacteria.

Another objective of the present disclosure is to provide a rigid disposable material which complies with the standard ISO 11607-1.

To accomplish this, the Applicant has succeeded in producing a cardboard whose characteristics allow it to achieve at least partially the objectives of the aforementioned standard and whose weight allows it to be transformed into trays or any other rigid receptacle.

More precisely, the present disclosure relates to a sterilizable fibrous material for the packaging of medical devices intended to be sterilized. The sterilizable fibrous material is characterized in that: it is in the form of a cardboard having a basis weight of at least 180 g/m², advantageously at least 200 g/m²; and it comprises a mixture of fibers containing at least 75% by weight of natural cellulose fibers, the length of which is less than 5 mm; and it has a permeability to air of at least 1.7, preferably at least 3.4, advantageously strictly greater than 3.4 µm/Pa.s (µm per Pascal second) at a pressure of 1.47 kPa, measured according to the ISO 5636-3 standard.

The greater the weight of the cardboard, the more rigid and easily transformable it is.

Thus, for example, the basis weight of the cardboard is advantageously at least 270 g/m², rendering it easily transformable, especially by thermoforming.

As previously mentioned, a major difficulty is to provide a sufficiently rigid material which is both permeable to the sterilization agent, such as steam, and barrier to bacteria.

In the present application, when reference is made to a standard, the applicable version is the one in force on the filing date of the priority application.

The Applicant has ascertained that particularly interesting performance is achieved in this regard when the mixture of fibers comprises: between 20% and 100% by weight of natural long cellulose fibers, the length of which is between 1 and 5 mm; and between 0% and 80% by weight of natural short cellulose fibers, the length of which is less than 1 mm.

The cellulose fibers are chosen, for example, from the group comprising pulp (short fibers and long fibers) and the fibers of annual plants, such as abaca, cotton, flax, and hemp.

Preferably, the long fibers have a length between 1 and 3 mm and preferably between 1.4 and 2.5 mm and the short fibers have a length between 0.2 and 1 mm, preferably between 0.3 and 1 mm.

In one particular embodiment, a portion of the cellulose fibers, preferably between 1 and 40% by weight, is treated with soda in order to form mercerized cellulose fibers.

The performance of the material according to the present disclosure can be further improved when the mixture contains long fibers and short fibers, the ratio of long fibers to short fibers being between 2 and 30.

In one preferred embodiment, the mixture of fibers contains between 70 and 80% of long fibers and between 15 and 25% of short cellulose fibers.

In order to increase the proportion of biosourced ingredients of the material of the present disclosure, the cellulose fibers represent at least 30% by weight, preferably at least 50% by weight, more preferably 70% by weight and even more preferably at least 90% by weight or even 95% by weight of the weight of the material.

In one particular embodiment, the mixture of fibers further comprises between at least 1% by weight, advantageously between 1 and 20% by weight, of chemical fibers.

In practice, the chemical fibers have a titer between 0.3 and 10 dtex and a length between 2.5 and 20 mm, preferably between 2.5 and 6 mm.

When present, the chemical fibers are advantageously chosen from the group comprising artificial fibers such as Lyocell and rayon, and synthetic fibers such as biopolymers like polylactic acid, polyhydroxyalcanoate, polybutylene succinates, polybutylene succinate co-adipates, polycaprolactones, polybutyrate adipate terephthalate, poly(hydroxybutyrate-co-hydroxyvalerate) or their copolymers.

The Applicant has observed that the selection of a mixture of lyocell fibers, of preferably 1.7 dtex and 6 mm and/or of rayon, in particular Danufil (1.7 dtex, 5 mm or 3 mm) made it possible to improve the permeability of the material.

Advantageously, the mixture of fibers is biosourced and/or recyclable and/or biodegradable.

Preferably, the material according to the present disclosure is biodegradable to a degree of 90%, or even 95%. Thus, the material meets the requirements of biodegradation of the standard EN 13432.

According to another characteristic and in certain embodiments, the material of the present disclosure has pores having a maximum pore diameter less than 50 µm, measured according to appendix C of standard EN 868-3. The material may also have a mean pore diameter also known as average pore diameter less than 35 µm, preferably between 10 µm and 35 µm, measured according to appendix C of standard EN 868-3. These characteristics thus allow it to comply with standard ISO 11607-1. The material can likewise comply with the standards of series EN 868. It is noted that when tested in accordance with Annex C of standard EN 868-3, the average of the pore diameters of the ten test pieces should be lower than or equal to 35 µm, and no pore diameter should be greater than 50 µm.

In practice, the material according to the present disclosure further comprises a wet strength agent, representing between 0.15 and 1% by dry weight with respect to the dry weight of cellulose, preferably on the order of 0.5%.

According to the present disclosure, the wet strength agent is chosen from the group comprising polyamine epichlorhydrin (PAE), glyoxalated resins, such as glyoxalated polyamide (GPAM), formaldehyde-based resin.

Preferably, the material according to the present disclosure further comprises a sizing agent representing preferably between 0.15 and 1% by dry weight in relation to the dry weight of cellulose, preferably on the order of 0.5%.

In practice, the sizing agent is chosen from the group comprising alkyl ketene dimer (AKD), alkenyl succinic anhydride (ASA), and rosin-based resin.

In one preferred embodiment, the material further comprises cationic starch representing less than 1% by dry weight in relation to the dry weight of cellulose, preferably on the order of 0.5%.

According to the present disclosure, the material has hydrophobic properties, preferably measured according to the standard ISO 535 using a COBB test at 60 seconds, of less than 20 g/m².

Advantageously, it complies with the standard DIN 58953-6 sections 3 and 4 in terms of bacterial barrier. The bacterial barrier properties can also be evaluated per the standard ASTM F2101. Thus, the material advantageously has a bacteria filtration efficacy (BFE) in a single layer greater than 80%, preferably greater than 95%, and more preferably greater than 99%.

As previously mentioned, it advantageously complies with the standard ISO 11607-1.

According to another characteristic, the material has a thickness of at least 200, preferably 300 µm, in order to give it optimal rigidity.

To render it heat-sealable, in particular when it is intended to be molded into a tray covered with a heat-sealable medical plastic film, said material further comprises a coating layer, the composition of which is able to render it heat-sealable.

The coating layer further allows limiting the surface roughness of the material and thus helps with the peelability of the film once it has been heat-sealed.

Advantageously, the coating contains at least one component chosen from among starch, polyvinylic alcohol (PVA), alkyl ketene dimer (AKD). The coating may further contain other components, such as bonding agents of the acrylic type, for example, or cross-linking agents, such as salts of zirconium and polyamine epichlorhydrin (PAE), for example.

In practice, the coating is applied at a rate of 4 to 30 g/m², preferably around 5 g/m².

In order to further improve the heat sealability, the material is calendered after the coating.

The present disclosure also relates to a tray or an equivalent receptacle obtained from the material described above.

The present disclosure also relates to the use of the previously described material for the fabrication of a tray.

Preferably, the tray is obtained by thermoforming.

In order to improve the thermoforming ability of the material of the present disclosure, it has a moisture level of at least 6% by weight.

In order to avoid the risks of tearing during the thermoforming, the elongation of the material in the machine direction (MD) and the cross-machine direction (CD), preferably determined according to the standard ISO 1924-2, is at least 3%.

The present disclosure also relates to a packaging for medical sterilization, comprising: a tray as previously described, or any equivalent means; and a means of tight sterilizable closure of the tray, such as a medical film of PET, PP or any other heat-sealable or gluable means.

### BRIEF DESCRIPTION OF THE DRAWINGS

Figure 1 is a picture of a tray of the present disclosure prior to sterilization, filled with a dye mixture.
Figure 2 is a picture of a tray of the present disclosure after sterilization, filled with a dye mixture.
Figure 3 is a picture of a tray of the present disclosure containing metallic parts after sterilization.
Figures 4A to 4D are pictures of samples of the present disclosure, and comparative samples, in a home composter.

### DETAILED DESCRIPTION OF THE DISCLOSURE

The exemplary embodiments disclosed herein are illustrative of advantageous sterilizable packages, and systems of the present disclosure and methods/techniques thereof. It should be understood, however, that the disclosed embodiments are merely exemplary of the present disclosure, which may be embodied in various forms. Therefore, details disclosed herein with reference to exemplary sterilizable packages and associated processes/techniques of assembly and use are not to be interpreted as limiting, but merely as the basis for teaching one skilled in the art how to make and use the advantageous sterilizable packages and/or alternative sterilizable packages of the present disclosure.

### Example 1: Composition of the samples

### a. Composition of the samples of the present disclosure

Four samples were fabricated, the compositions of which are given in Table 1:

**Table 1:**

| | | Sample 1 | Sample 2 | Sample 3 | Sample 4 | Sample 5 | Sample 6 |
|---|---|---|---|---|---|---|---|
| Mixture of fibers | Sodra Blue Z | 76% | 72% | 76% | 72% | 72% | 32% |
| | Navia | 19% | 18% | 19% | 18% | 18% | 16% |
| | Botnia Nordic | | | | | | 32% |
| | Buckeye HPZ | | | | | | 20% |
| | Danufil 1.7 dtex - 5mm | 5% | 10% | | | | |
| | Danufil 1.7 dtex - 3mm | | | | | 10% | |
| | Lyocell 1.7 dtex - 5mm | | | 5% | 10% | | |
| | | | | | | | |
| Wet strength agent (in dry weight of cellulose) | Kymene 20 Xcel | 0.4 | 0.4 | 0.4 | 0.4 | 0.4 | 0.4 |
| Starch | Hi Cat 1134A | 0.5 | 0.5 | 0.5 | 0.5 | 0.5 | 0.5 |
| Sizing agent | Fennosize 860D | 0.5 | 0.5 | 0.5 | 0.5 | 0.5 | 0.5 |
| Coating (g/m²)° | | 4.3 with 90% PVA, 5% AKD and 5% Kymene | 5.4 with 90% PVA, 5% AKD and 5% Kymene | 4.5 with 90% PVA, 5% AKD and 5% Kymene | 5.2 with 90% PVA, 5% AKD and 5% Kymene | 7 with 61% PVA, 7% AKD, 30.5 acrylic latex, 1.5% zirconium salt | 27 with 63% PVA, 4% AKD, 32% acrylic latex, 1% zirconium salt |

### b. COMPARISON SAMPLE

This was a spunbond composed of fibers of polypropylene and polyolefin with a basis weight of 90.5 g/m² marketed by AMCOR 095134 under the brand name ULTRA^{®}.

### EXAMPLE 2: Characteristics of the samples

The major characteristics of the different samples are given in Table 2.

**Table 2:**

| Test | Unit | EN 868-3 | Sample 1 | Sample 2 | Sample 3 | Sample 4 | Sample 5 | Sample 6 | Comp. sample |
|---|---|---|---|---|---|---|---|---|---|
| Weight ISO 536 | g/m² | | 297 | 275 | 278 | 293 | 284 | 332 | 90.5 |
| Thickness ISO 534 | µm | | 466 | 474 | 450 | 485 | 441 | 476 | 174 |
| Permeability to air ISO 5636-3 | ml/ min | >400 ml/mi n at 1.47 kPa | 655 | 1332 | 650 | 1104 | 2014 | 974 | 1696 |
| Cobb (60 s) ISO 535 | g/m² | <20 g/m² | 19.66 | 19.64 | 19.4 | 19.66 | 18.7 | 17 | 2.8 |
| Pore diameter EN 868-3 Appendix C | µm | <=35 (max 50) | 12.6 (18) | 15.9 (22.6) | 14.1 (19.3) | 15.2 (21.5) | 14.6 (21) | 15 (19.5) | 26 (41) |

Samples 1 to 5 were compliant with the standard ISO 11607-1.

### EXAMPLE 3: Characteristics Of The Sealing Of The Tray According To The Present Disclosure

### a. By a Brugger test

Sample 4 was calendered (30 kN/m at 50°C) and subjected to a heat sealing test with a medical film using Brugger clamps at 180° C with a force of 4 bars for 10 seconds. No tear-off of fibers was observed and the seal showed a value greater than 1.5 N/15 mm.

### b. By injection of a dye per standard ASTM F 1929

### i. Quality of the seal in absence of sterilization

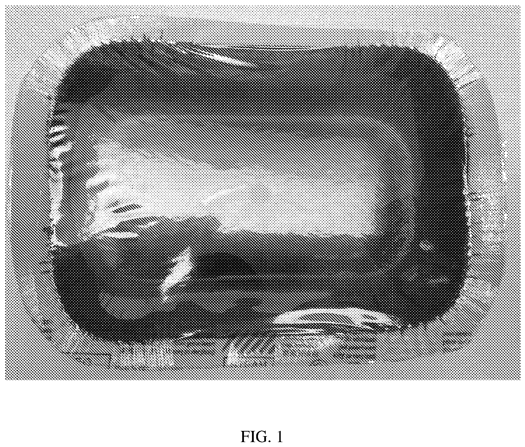

Sample 5 was thermoformed to produce a tray. The tray was sealed with a medical film for sterilization by steam or ethylene oxide made of PP/PET. An aqueous solution with a blue dye and a surfactant was injected in the tray. The colored solution was moved to the area of the sealing points on each side and maintained for 5 seconds each time, for a total duration of 20 seconds per the standard ASTM F 1929. No leak was observed (see FIG. 1). Following the standard EN 868-5, calendered sample 6 was subjected to a heat sealing test as with a medical film using Brugger clamps at 190°C with a force of 5 bars for respectively 2, 3, 4 and 5 seconds. All samples had a peel strength greater than 1.5 N/15 mm but remained less than 5 N/15 mm. This showed that a tray according to the present disclosure can be properly sealed with a medical film and be opened without significant effort and without fibers tearing. As shown in FIG. 1, the tray extends from a first end to a second end (from left to right), and extends from a first side to a second side (from the top of FIG. 1 to the bottom of FIG. 1). The first end includes a first end wall, and the second end includes a second end wall. The first side includes a first side wall, and the second side includes a second side wall. The first and second end walls and the first and second side walls define a recess or cavity within the internal boundaries of the first and second end walls and the first and second side walls (e.g., with the recess/cavity housing the dye mixture), with a bottom wall at the bottom of the recess/cavity.

### ii. Quality of the seal after sterilization

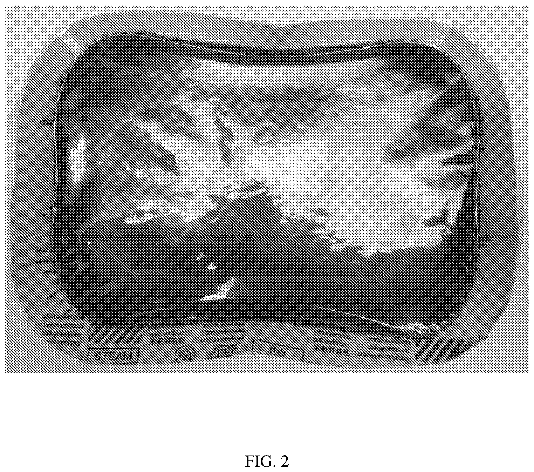

Sample 5 was thermoformed to produce a tray. The tray was then sealed with a medical film for sterilization by steam or ethylene oxide made of PP/PET, and then sterilized with steam for 18 min at 134° C. An aqueous solution with a blue dye and a surfactant was then injected in the tray. The colored solution was moved to the area of the sealing points on each side and maintained for 5 seconds each time, for a total duration of 20 seconds per the standard ASTM F 1929. No leak was observed (see FIG. 2).

### Example 4: Test for evaluation of microbiological contamination of a tray according to the present disclosure

The purpose of this test was to show the ability of the material of the present disclosure to let the sterilizing steam pass through it, thus allowing a sterilization of the elements contained in it.

To do this, a tray was fabricated from sample 5. On this tray was arranged 22 metallic pieces contaminated with microorganisms contained in saliva. The tray was then heat sealed with a film for sterilization by steam or ethylene oxide made of PP/PET. The tray was then sterilized with steam at 134 °C for 18 minutes. The tray was stored under normal conditions of pressure and temperature for 15 days prior to microbial analysis of 3 metallic pieces, chosen at random from the 22 pieces.

The microbial contamination was evaluated by the method ISO 8784-1 and was based on a counting of bacteria and fungi. The medium used to culture the bacteria was Tryptic Soy Agar with an incubation of 48 h at 37 °C. The medium used to culture the fungi was PDA with an incubation of 5 days at 29 °C.

The results are shown in Table 3:

**Table 3:**

| Sample | N of fungi | N of bacteria |
|---|---|---|
| Metallic piece 1 | <10 CFU/part | <10 CFU/part |
| Metallic piece 2 | <10 CFU/part | <10 CFU/part |
| Metallic piece 3 | <10 CFU/part | <10 CFU/part |

| | | |
|---|---|---|
| CFU: colony forming unit N: total number of microorganisms calculated in UFC per piece | | |

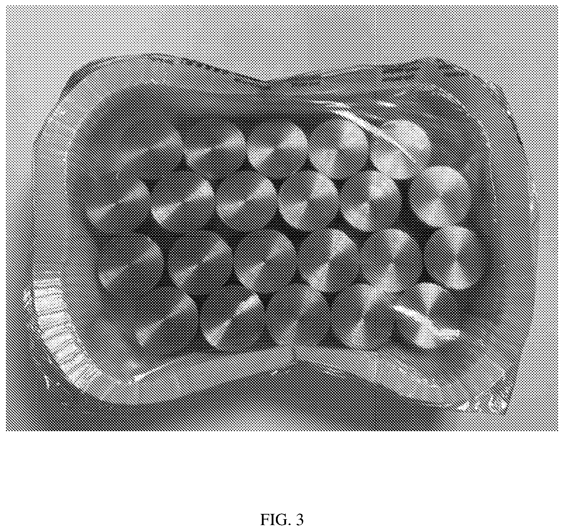

The results show that, after sterilization, the metallic pieces are no longer contaminated, which proves that the material of the present disclosure is permeable to the sterilizing steam and remains sterile after a storage at room temperature for 15 days. This test also showed the mechanical strength of the tray (see FIG. 3). In fact, it was possible to sterilize 22 metallic pieces weighing more than 1 kg and store them in sterile manner without any tearing or opening.

### Example 5: Test for bacterial resistance of the material of the present disclosure

### a. In humid conditions

The test was performed on the 4 samples (samples 1-4) according to the present disclosure and one comparison sample under the conditions of the standard DIN 58953-6, section 3. Basically, the test involved sterilizing each sample with steam at 134 °C for 4 minutes. Each sample was then inoculated with 500 µL of the microbe *S*. *epidermidis* on one surface. After drying, the opposite surface of the sample was placed in contact with a culture medium and incubated for 24h at 37 °C. The two surfaces of each sample were tested.

The results are shown in Table 4.

**Table 4: Test for bacteria resistance in humid conditions per standard DIN 58953-6, section 3**

| Sample | Surface | Number of CFU/agar plate | | | | | |
|---|---|---|---|---|---|---|---|
| | | 1 | 2 | 3 | 4 | 5 | ∑ |
| Comparison sample (Amcor ultra white spunbond 90 g/m² | A | 0 | 0 | 0 | 0 | 0 | 0 |
| | B | 0 | 0 | 0 | 0 | 0 | 0 |
| Sample 1 | A | 0 | 0 | 0 | 0 | 0 | 0 |
| | B | 0 | 0 | 0 | 0 | 0 | 0 |
| Sample 2 | A | 0 | 0 | 0 | 0 | 0 | 0 |
| | B | 0 | 0 | 0 | 0 | 0 | 0 |
| Sample 3 | A | 0 | 0 | 0 | 0 | 0 | 0 |
| | B | 0 | 0 | 0 | 0 | 0 | 0 |
| Sample 4 | A | 0 | 0 | 0 | 0 | 0 | 0 |
| | B | 0 | 0 | 0 | 0 | 0 | 0 |
| Sample 6 | A | 0 | 0 | 0 | 0 | 0 | 0 |
| | B | 0 | 0 | 0 | 0 | 0 | 0 |

| | | | | | | | |
|---|---|---|---|---|---|---|---|
| CFU= colony forming unit | | | | | | | |

No colony was found on any of the samples of the present disclosure or the comparison sample. The test for bacteria resistance in humid conditions according to standard DIN 58953-6, section 3 was thus validated.

### b. In dry conditions

### i. Standard DIN 58953-6, section 4

The test was performed on 4 samples (samples 1-4) according to the present disclosure and one comparison sample under the conditions of the standard DIN 58953-6, section 4. Basically, the test involved sterilizing each sample with steam at 121 °C for 20 minutes. Each sample was then inoculated with 250 mg of sand contaminated with endospores of *B. subtilis.* Incubations were then done at 8 °C and 50 °C to create a flow of air through the contaminated sand and the sample as far as the agar plate. The samples were then incubated for 24h at 37 °C. The 2 surfaces of each sample were tested.

**Table 5: Test for bacteria resistance in dry conditions per standard DIN 58953-6, section 4**

| Sample | Surface | Number of CFU/agar plate | | | | | |
|---|---|---|---|---|---|---|---|
| | | 1 | 2 | 3 | 4 | 5 | ∑ |
| Comparison sample (Amcor ultra white spunbond 90 g/m² | A | 0 | 0 | 0 | 0 | 0 | 0 |
| | B | 0 | 0 | 0 | 0 | 0 | 0 |
| Sample 1 | A | 0 | 0 | 0 | 0 | 0 | 0 |
| | B | 0 | 0 | 0 | 0 | 0 | 0 |
| Sample 2 | A | 0 | 0 | 0 | 0 | 0 | 0 |
| | B | 0 | 0 | 0 | 0 | 0 | 0 |
| Sample 3 | A | 0 | 0 | 0 | 0 | 0 | 0 |
| | B | 0 | 0 | 0 | 0 | 0 | 0 |
| Sample 4 | A | 0 | 0 | 0 | 0 | 0 | 0 |
| | B | 0 | 0 | 0 | 0 | 0 | 0 |
| Sample 6 | A | 0 | 0 | 0 | 0 | 0 | 0 |
| | B | 0 | 0 | 0 | 0 | 0 | 0 |

| | | | | | | | |
|---|---|---|---|---|---|---|---|
| CFU= colony forming unit | | | | | | | |

As expected, no colony was found on any of the samples of the present disclosure or the comparison sample. This was expected, inasmuch as the test in humid environment according to standard DIN 58953-6 section 3 is challenging, water serving as the vector of contamination. The test for bacteria resistance under dry conditions according to standard DIN 58953-6, section 4 was thus validated.

### ii. Bacterial filtration efficiency (BFE)

This test was performed according to the standard ASTM F2101, using an aerosol of *Staphylococcus aureus.* This test expresses the ratio of the number of bacteria arrested per sample, divided by the number of bacteria sprayed onto the sample tested.

### Table 6:

| Sample 5 | Bacterial filtration efficiency (BFE) % | Comparison sample, 1 sheet | Bacterial filtration efficiency (BFE) % | Comparison sample, 2 sheets | Bacterial filtration efficiency (BFE) % |
|---|---|---|---|---|---|
| 1 | 99.9 | 1 | 76.8 | 1 | 93.6 |
| 2 | 99.9 | 2 | 71.0 | 2 | 91.0 |
| 3 | 99.9 | 3 | 71.5 | 3 | 92.0 |
| 4 | 99.9 | 4 | 73.4 | 4 | 94.3 |
| 5 | 99.9 | 5 | 63.5 | 5 | 94.9 |

As shown by Table 6, sample 5 has a BFE of 99.9% as compared to the comparison sample, which has a BFE of 71.24% for a packaging of 1 sheet and 93.16% for a packaging of 2 sheets. The sample according to the present disclosure presents a more tortuous pathway for the microorganisms and thus minimizes the risks of contamination.

Breathability is the ability of a material to allow water vapor to pass through it while preventing liquid water from passing through it, and is expressed interchangeably as "water vapor transmission rate" (WVTR), or "moisture vapor transmission rate" (MVTR). Accordingly, the material of the present disclosure has an overall water vapor transmission rate (WVTR) of at least 400 g/m²/day, preferably at least 800 g/m²/day, and more preferably at least 1000 g/m²/day at 38°C and 90 % relative humidity and as determined by the ISO 2528 standard.

### Example on MVTR and Bowie and Dick test:

The WVTR of sample 6 and comparative samples were measured according to the ISO 2528 standard, and the results are summarized in Table 7.

**Table 7:**

| Samples | WVTR (g/m²/day) |
|---|---|
| Sample 6 (single layer) | 2071 |
| Sample 6 (double layer) | 1698 |
| Comparative sample (single layer) | 2146 |
| Comparative sample (single layer) | 1492 |

The samples were measured following the ISO 2528 standard and at 38 °C and 90% relative humidity. The data shows that sample 6 has comparable WVTR to the comparative sample, while the basis weight of sample 6 is more than triple of the comparative sample. The material according to the present disclosure can thus be efficiently sterilized.

In order to verify the uniformity and efficiency of the vapor penetration, sample 6 was thermoformed into a tray and a Bowie and Dick test kit was placed in the tray. The tray was then sealed with a medical film and was subjected to a sterilization cycle (Bowie and Dick cycle: 3.5 minutes at 134°C). After sterilization, the Bowie and Dick kit test showed efficient and uniform steam penetration.

### Biodegradability and Compostability

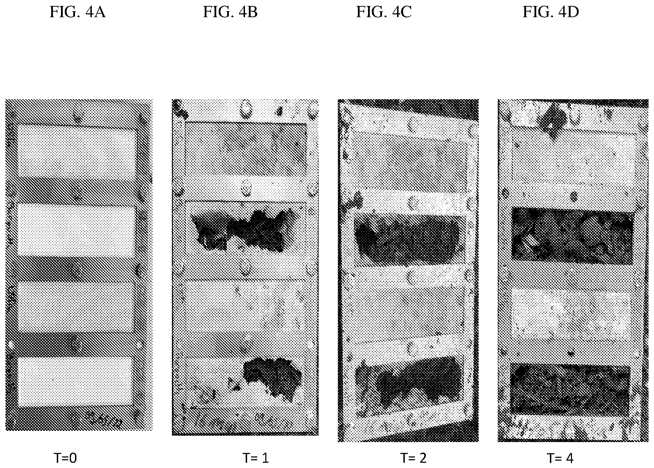

Two of each sample 6 (second from top row, and bottom row of FIGS. 4A to 4D) and comparative samples (top row, and one above the bottom row of FIGS. 4A to 4D) were placed in frame and were introduced in a home composter. The samples were then visually inspected after one, two weeks and four weeks. As it can be seen in FIGS. 4A to 4D, sample 6 has almost completely biodegraded after four weeks, whereas the comparative samples remained intact.

### Stiffness and Rigidity

In order to form a rigid tray, the material according to the present disclosure had a higher stiffness compared to other sterilization materials such as pouches or sterilization wraps. The rigidity or stiffness of a material may be measured by determining the resistance to bending of the material following ISO 2493-2 - Paper and Board - Determination of resistance to bending - part 2 : Taber Type tester Standard. The material according to the present disclosure may have a resistance to bending of at least 1000 mN in MD and CD directions, preferably at least 2000 mN and even more preferably at least 3000 mN.

### Example On Resistance To Bending

The resistance to bending of Sample 6 and comparative sample were measured in the machine direction (MD) and the cross-machine direction (CD) (see Table 8, below) following the ISO 2493-2 standard.

**Table 8:**

| | Resistance in MD direction (mN) | Resistance in CD direction (mN) |
|---|---|---|
| Sample 6 | 6292 | 4938 |
| Comparative Sample | 282 | 238 |

The Comparative sample had a basis weight of 90 g/m² and sample 6 had a basis weight of 332 g/m² but the material according to the present disclosure had a much higher resistance to bending. Such resistance makes sample 6 suitable for making a rigid tray according to the present disclosure.

## Claims

1. A sterilizable fibrous material for the packaging of medical devices intended to be sterilized, **characterized in that**:
the sterilizable fibrous material is in the form of a cardboard having a basis weight of at least 180 g/m², advantageously at least 200 g/m²; and
the sterilizable fibrous material comprises a mixture of fibers containing at least 75% by weight of natural cellulose fibers, the length of which is less than 5 mm; and
the sterilizable fibrous material has a permeability to air of at least 1.7, preferably at least 3.4, advantageously strictly greater than 3.4 µm/Pa.s at a pressure of 1.47 kPa, measured according to the ISO 5636-3 standard; and
the sterilizable fibrous material has pores having a maximum pore diameter less than 50 µm, measured according to appendix C of standard EN 868-3.

2. The material according to claim 1, **characterized in that** the mixture of fibers contains:
between 20% and 100% by weight of natural long cellulose fibers, the length of which is between 1 and 5 mm; and
between 0% and 80% by weight of natural short cellulose fibers, the length of which is less than 1 mm.

3. The material according to any one of the preceding claims, **characterized in that** the long fibers have a length between 1 and 3 mm, and preferably between 1.4 and 2.5 mm, and the short fibers have a length between 0.2 and 1 mm, preferably between 0.3 and 1 mm.

4. The material according to any one of the preceding claims, **characterized in that** the ratio of long fibers to short fibers is between 2 and 30.

5. The material according to any one of the preceding claims, **characterized in that** the mixture of fibers further comprises between at least 1% by weight, preferably between 1 and 20% by weight, of chemical fibers having a titer between 0.3 and 10 dtex and a length between 2.5 and 20 mm.

6. The material according to any one of the preceding claims, **characterized in that** the chemical fibers are chosen from the group comprising artificial fibers such as Lyocell and rayon, and synthetic fibers such as biopolymers like polylactic acid, polyhydroxyalcanoate, polybutylene succinates, polybutylene succinate co-adipates, polycaprolactones, polybutyrate adipate terephthalate, poly(hydroxybutyrate-co-hydroxyvalerate) or their copolymers.

7. The material according to any one of the preceding claims, **characterized in that** the mixture of fibers is biosourced and/or recyclable and/or biodegradable.

8. The material according to any one of the preceding claims, **characterized in that**:
it is biodegradable to a degree of 90%, or even 95% and meets the requirements of biodegradation of the standard EN 13432; and/or
it has pores having a mean pore diameter less than 35 µm preferably between 10 µm and 35 µm, measured according to appendix C of standard EN 868-3; and/or
it has a COBB at 60 seconds, measured according to the standard ISO 535, of less than 20 g/m²; and/or
it complies with the standard DIN 58953-6 sections 3 and 4 in terms of a bacterial barrier; and/or
it complies with the standard ISO 11607-1; and/or
it has a thickness of at least 200, preferably 300 µm; and/or
it is calendered; and/or
the material has a resistance to bending of at least 1000 mN in a machine direction and a cross-machine direction, preferably at least 2000 mN, and even more preferably at least 3000 mN, according to ISO 2493-2, Paper and Board, Determination of resistance to bending, part 2: Taber Type tester Standard; and/or
the material has an overall water vapor transmission rate (WVTR) of at least 400 g/m²/day, preferably at least 800 g/m²/day, and more preferably at least 1000 g/m²/day at 38°C and 90 % relative humidity and as determined by the ISO 2528 standard.

9. The material according to any one of the preceding claims, **characterized in that** it further comprises a wet strength agent, representing between 0.15 and 1% by dry weight with respect to the dry weight of cellulose.

10. The material according to claim 9, **characterized in that** the wet strength agent is chosen from the group comprising polyamine epichlorhydrin (PAE), glyoxalated resins, such as glyoxalated polyamide (GPAM), formaldehyde-based resin.

11. The material according to any one of the preceding claims, **characterized in that** it further comprises a sizing agent representing between 0.15 and 1% by dry weight in relation to the dry weight of cellulose.

12. The material according to claim 11, **characterized in that** the sizing agent is chosen from the group comprising alkyl ketene dimer (AKD), alkenyl succinic anhydride (ASA), and rosin-based resin.

13. The material according to any one of the preceding claims, **characterized in that** it further comprises a coating layer, the composition of which is able to render it heat-sealable.

14. The material according to any one of the preceding claims, **characterized in that** the mixture of fibers further comprises between 1 and 40% by weight mercerized cellulose fibers.

15. Tray obtained from the material specified by any one of claims 1 to 14.

16. Use of the material specified by any one of claims 1 to 14 for the fabrication of a tray.

17. Packaging for medical sterilization, comprising:
a tray according to claim 15; and
a means of tight sterilizable closure of the tray.

18. A container for the packaging of medical devices intended to be sterilized comprising:
a tray comprising a sterilizable fibrous material, the tray extending from a first end to a second end, and extending from a first side to a second side, the first end including a first end wall and the second end including a second end wall, and the first side including a first side wall and the second side including a second side wall, with the first and second end walls and the first and second side walls defining a recess or cavity within internal boundaries of the first and second end walls and the first and second side walls, and with a bottom wall at a bottom of the recess or cavity;
wherein the tray has a basis weight of at least 180 g/m², advantageously at least 200 g/m²; and
wherein the tray comprises a mixture of fibers containing at least 75% by weight of natural cellulose fibers, the length of which is less than 5 mm; and
wherein the tray has a permeability to air of at least 1.7, preferably at least 3.4, advantageously strictly greater than 3.4 µm/Pa.s at a pressure of 1.47 kPa, measured according to the ISO 5636-3 standard; and
wherein the tray has pores having a maximum pore diameter less than 50 µm, measured according to appendix C of standard EN 868-3.

19. A method for fabricating a container for the packaging of medical devices intended to be sterilized comprising:
providing a sterilizable fibrous material; and
forming the sterilizable fibrous material in to a tray, the tray extending from a first end to a second end, and extending from a first side to a second side, the first end including a first end wall and the second end including a second end wall, and the first side including a first side wall and the second side including a second side wall, with the first and second end walls and the first and second side walls defining a recess or cavity within internal boundaries of the first and second end walls and the first and second side walls, and with a bottom wall at a bottom of the recess or cavity;
wherein the tray has a basis weight of at least 180 g/m², advantageously at least 200 g/m²; and
wherein the tray comprises a mixture of fibers containing at least 75% by weight of natural cellulose fibers, the length of which is less than 5 mm; and
wherein the tray has a permeability to air of at least 1.7, preferably at least 3.4, advantageously strictly greater than 3.4 µm/Pa.s at a pressure of 1.47 kPa, measured according to the ISO 5636-3 standard; and
wherein the tray has pores having a maximum pore diameter less than 50 µm, measured according to appendix C of standard EN 868-3.

20. The method of claim 19, wherein forming the sterilizable fibrous material in to the tray comprises thermoforming the sterilizable fibrous material or molding the sterilizable fibrous material.

## Patentansprüche

1. Sterilisierbares Fasermaterial für die Verpackung von medizinischen Vorrichtungen, die sterilisiert werden sollen, **dadurch gekennzeichnet, dass**:
das sterilisierbare Fasermaterial die Form eines Kartons mit einem Flächengewicht von mindestens 180 g/m², vorteilhafterweise von mindestens 200 g/m², aufweist; und
das sterilisierbare Fasermaterial eine Fasermischung umfasst, die mindestens 75 Gewichts-% natürliche Cellulosefasern, deren Länge weniger als 5 mm beträgt, enthält; und
das sterilisierbare Fasermaterial eine Luftdurchlässigkeit von mindestens 1,7, vorzugsweise mindestens 3,4, vorteilhafterweise strikt größer als 3,4 µm/Pa.s bei einem Druck von 1,47 kPa, gemessen gemäß der ISO 5636-3 Norm, aufweist; und
das sterilisierbare Fasermaterial Poren mit einem maximalen Porendurchmesser von weniger als 50 µm, gemessen gemäß Anhang C der EN 868-3 Norm, aufweist.

2. Material gemäß Anspruch 1, **dadurch gekennzeichnet, dass** die Fasermischung folgendes enthält:
zwischen 20 und 100 Gewichts-% natürliche lange Cellulosefasern, deren Länge zwischen 1 und 5 mm liegt; und
zwischen 0 und 80 Gewichts-% natürliche kurze Cellulosefasern, deren Länge weniger als 1 mm beträgt.

3. Material gemäß mindestens einem der vorhergehenden Ansprüche, **dadurch gekennzeichnet, dass** die langen Fasern eine Länge zwischen 1 und 3 mm, vorzugsweise zwischen 1,4 und 2,5 mm, und die kurzen Fasern eine Länge zwischen 0,2 und 1 mm, vorzugsweise zwischen 0,3 und 1 mm, aufweisen.

4. Material gemäß mindestens einem der vorhergehenden Ansprüche, **dadurch gekennzeichnet, dass** das Verhältnis von langen Fasern zu kurzen Fasern zwischen 2 und 30 liegt.

5. Material gemäß mindestens einem der vorhergehenden Ansprüche, **dadurch gekennzeichnet, dass** die Fasermischung ferner mindestens 1 Gewichts-%, vorzugsweise zwischen 1 und 20 Gewichts-%, chemische Fasern mit einem Titer zwischen 0,3 und 10 dtex und einer Länge zwischen 2,5 und 20 mm umfasst.

6. Material gemäß mindestens einem der vorhergehenden Ansprüche, **dadurch gekennzeichnet, dass** die chemischen Fasern ausgewählt sind aus der Gruppe umfassend Kunstfasern wie Lyocell und Rayon und synthetische Fasern wie Biopolymere wie Polymilchsäure, Polyhydroxyalcanoat, Polybutylensuccinate, Polybutylensuccinat-Coadipate, Polycaprolactone, Polybutyrat-Adipat-Terephthalat, Poly(hydroxybutyrat-co-hydroxyvalerat) oder deren Copolymere.

7. Material gemäß mindestens einem der vorhergehenden Ansprüche, **dadurch gekennzeichnet, dass** die Fasermischung aus biologischem Anbau stammt und/oder recycelbar und/oder biologisch abbaubar ist.

8. Material gemäß mindestens einem der vorhergehenden Ansprüche, **dadurch gekennzeichnet, dass**:
es zu einem Grad von 90 % oder sogar 95 % biologisch abbaubar ist und die Anforderungen der EN 13432 Norm an die biologische Abbaubarkeit erfüllt; und/oder
es Poren mit einem mittleren Porendurchmesser von weniger als 35 µm, vorzugsweise zwischen 10 µm und 35 µm, gemessen gemäß Anhang C der EN 868-3 Norm, aufweist; und/oder
es einen COBB-Wert bei 60 Sekunden von weniger als 20 g/m² aufweist, gemessen nach der ISO 535 Norm; und/oder
es den Abschnitten 3 und 4 der DIN 58953-6 Norm in Bezug auf eine bakterielle Barriere entspricht; und/oder
es der ISO 11607-1 Norm entspricht; und/oder es eine Dicke von mindestens 200, vorzugsweise 300 µm aufweist; und/oder
es kalandriert ist; und/oder
das Material eine Biegefestigkeit von mindestens 1000 mN in Maschinenrichtung und quer zur Maschinenrichtung, vorzugsweise mindestens 2000 mN und mehr bevorzugt mindestens 3000 mN, gemäß ISO 2493-2, Papier und Pappe, Bestimmung der Biegefestigkeit, Teil 2: Taber Type tester Standard, aufweist; und/oder
das Material eine Gesamtwasserdampfdurchlässigkeitsrate (WVTR) von mindestens 400 g/m²/Tag, vorzugsweise mindestens 800 g/m²/Tag und besonders bevorzugt mindestens 1000 g/m²/Tag bei 38°C und 90 % relativer Luftfeuchtigkeit und bestimmt gemäß der ISO 2528 Norm aufweist.

9. Material gemäß mindestens einem der vorhergehenden Ansprüche, **dadurch gekennzeichnet, dass** es ferner ein Nassfestigkeitsmittel umfasst, das zwischen 0,15 und 1 % des Trockengewichts, bezogen auf das Trockengewicht der Cellulose, ausmacht.

10. Material gemäß Anspruch 9, **dadurch gekennzeichnet, dass** das Nassfestigkeitsmittel ausgewählt ist aus der Gruppe umfassend Polyaminepichlorhydrin (PAE), glyoxalierte Harze, wie glyoxaliertes Polyamid (GPAM), Harz auf Formaldehydbasis.

11. Material gemäß mindestens einem der vorhergehenden Ansprüche, **dadurch gekennzeichnet, dass** es ferner ein Schlichtemittel umfasst, das zwischen 0,15 und 1 % des Trockengewichts, bezogen auf das Trockengewicht der Cellulose, ausmacht.

12. Material gemäß Anspruch 11, **dadurch gekennzeichnet, dass** das Schlichtemittel ausgewählt ist aus der Gruppe umfassend Alkylketendimer (AKD), Alkenylsuccinanhydrid (ASA) und Harz auf Kolophoniumbasis.

13. Material gemäß mindestens einem der vorhergehenden Ansprüche, **dadurch gekennzeichnet, dass** es ferner eine Überzugsschicht umfasst, deren Zusammensetzung es heißsiegelfähig machen kann.

14. Material gemäß mindestens einem der vorangehenden Ansprüche, **dadurch gekennzeichnet, dass** die Fasermischung ferner zwischen 1 und 40 Gewichts-% mercerisierte Cellulosefasern umfasst.

15. Schale, hergestellt aus dem in mindestens einem der Ansprüche 1 bis 14 angegebenen Material.

16. Verwendung des in mindestens einem der Ansprüche 1 bis 14 angegebenen Materials zur Herstellung einer Schale.

17. Verpackung für die medizinische Sterilisation, umfassend:
eine Schale gemäß Anspruch 15; und
ein Mittel zum dichten sterilisierbaren Verschließen des Tabletts.

18. Behälter für die Verpackung von medizinischen Vorrichtungen, die sterilisiert werden sollen, umfassend:
eine Schale, die ein sterilisierbares Fasermaterial umfasst, wobei sich die Schale von einem ersten Ende zu einem zweiten Ende und von einer ersten Seite zu einer zweiten Seite erstreckt, wobei das erste Ende eine erste Endwand und das zweite Ende eine zweite Endwand einschließt und die erste Seite eine erste Seitenwand und die zweite Seite eine zweite Seitenwand einschließt, wobei die erste und die zweite Endwand und die erste und die zweite Seitenwand eine Aussparung oder einen Hohlraum innerhalb der inneren Begrenzungen der ersten und der zweiten Endwand und der ersten und der zweiten Seitenwand definieren, und mit einer Bodenwand an einem Boden der Aussparung oder des Hohlraums;
wobei die Schale ein Flächengewicht von mindestens 180 g/m², vorteilhafterweise von mindestens 200 g/m², aufweist; und
wobei die Schale eine Fasermischung umfasst, die mindestens 75 Gewichts-% natürliche Cellulosefasern, deren Länge weniger als 5 mm beträgt, enthält; und
wobei die Schale eine Luftdurchlässigkeit von mindestens 1,7, vorzugsweise mindestens 3,4, vorteilhafterweise strikt größer als 3,4 µm/Pa.s bei einem Druck von 1,47 kPa, gemessen gemäß der ISO 5636-3 Norm, aufweist; und
wobei die Schale Poren mit einem maximalen Porendurchmesser von weniger als 50 µm, gemessen gemäß Anhang C der EN 868-3 Norm, aufweist.

19. Verfahren zur Herstellung eines Behälters für die Verpackung von medizinischen Vorrichtungen, die sterilisiert werden sollen, umfassend:
Bereitstellen eines sterilisierbaren Fasermaterials; und
Formen des sterilisierbaren Fasermaterials zu einer Schale, wobei sich die Schale von einem ersten Ende zu einem zweiten Ende und von einer ersten Seite zu einer zweiten Seite erstreckt, wobei das erste Ende eine erste Endwand und das zweite Ende eine zweite Endwand einschließt und die erste Seite eine erste Seitenwand und die zweite Seite eine zweite Seitenwand einschließt, wobei die erste und die zweite Endwand und die erste und die zweite Seitenwand eine Aussparung oder einen Hohlraum innerhalb der inneren Begrenzungen der ersten und der zweiten Endwand und der ersten und der zweiten Seitenwand definieren, und mit einer Bodenwand an einem Boden der Aussparung oder des Hohlraums;
wobei die Schale ein Flächengewicht von mindestens 180 g/m², vorteilhafterweise von mindestens 200 g/m², aufweist; und
wobei die Schale eine Fasermischung umfasst, die mindestens 75 Gewichts-% natürliche Cellulosefasern, deren Länge weniger als 5 mm beträgt, enthält; und
wobei die Schale eine Luftdurchlässigkeit von mindestens 1,7, vorzugsweise mindestens 3,4, vorteilhafterweise strikt größer als 3,4 µm/Pa.s bei einem Druck von 1,47 kPa, gemessen gemäß der ISO 5636-3 Norm, aufweist; und
wobei die Schale Poren mit einem maximalen Porendurchmesser von weniger als 50 µm, gemessen gemäß Anhang C der EN 868-3 Norm, aufweist.

20. Verfahren gemäß Anspruch 19, bei dem das Formen des sterilisierbaren Fasermaterials zu der Schale Thermoformen des sterilisierbaren Fasermaterials oder Gießen des sterilisierbaren Fasermaterials umfasst.

## Revendications

1. Matériau fibreux stérilisable pour l'emballage de dispositifs médicaux destinés à être stérilisés, **caractérisé en ce que** :
le matériau fibreux stérilisable est sous la forme d'un carton ayant un poids de base d'au moins 180 g/m², avantageusement d'au moins 200 g/m² ; et
le matériau fibreux stérilisable comprend un mélange de fibres contenant au moins 75 % en poids de fibres de cellulose naturelle, dont la longueur est inférieure à 5 mm ; et
le matériau fibreux stérilisable a une perméabilité à l'air d'au moins 1,7, de préférence d'au moins 3,4, avantageusement strictement supérieure à 3,4 µm/Pa.s à une pression de 1,47 kPa, mesurée selon la norme ISO 5636-3 ; et
le matériau fibreux stérilisable a des pores ayant un diamètre de pore maximal inférieur à 50 µm, mesuré selon l'annexe C de la norme EN 868-3.

2. Matériau selon la revendication 1, **caractérisé en ce que** le mélange de fibres contient :
entre 20 % et 100 % en poids de fibres de cellulose naturelle longues, dont la longueur est comprise entre 1 et 5 mm ; et
entre 0 % et 80 % en poids de fibres de cellulose naturelle courtes, dont la longueur est inférieure à 1 mm.

3. Matériau selon l'une quelconque des revendications précédentes, **caractérisé en ce que** les fibres longues ont une longueur comprise entre 1 et 3 mm, et de préférence entre 1,4 et 2,5 mm, et les fibres courtes ont une longueur comprise entre 0,2 et 1 mm, de préférence entre 0,3 et 1 mm.

4. Matériau selon l'une quelconque des revendications précédentes, **caractérisé en ce que** le rapport des fibres longues aux fibres courtes est compris entre 2 et 30.

5. Matériau selon l'une quelconque des revendications précédentes, **caractérisé en ce que** le mélange de fibres comprend en outre entre au moins 1 % en poids, de préférence entre 1 et 20 % en poids, de fibres chimiques ayant un titre compris entre 0,3 et 10 dtex et une longueur comprise entre 2,5 et 20 mm.

6. Matériau selon l'une quelconque des revendications précédentes, **caractérisé en ce que** les fibres chimiques sont choisies dans le groupe comprenant les fibres artificielles telles que Lyocell et la rayonne, et les fibres synthétiques telles que les biopolymères comme l'acide polylactique, le polyhydroxyalcanoate, les polybutylène succinates, les polybutylène succinate co-adipates, les polycaprolactones, le polybutyrate adipate téréphtalate, le poly (hydroxybutyrate-co-hydroxyvalérate) ou leurs copolymères.

7. Matériau selon l'une quelconque des revendications précédentes, **caractérisé en ce que** le mélange de fibres est biosourcé et/ou recyclable et/ou biodégradable.

8. Matériau selon l'une quelconque des revendications précédentes, **caractérisé en ce que** :
il est biodégradable à un degré de 90 %, voire 95 % et satisfait les exigences de biodégradation de la norme EN 13432 ; et/ou
il a des pores ayant un diamètre de pore moyen inférieur à 35 µm, de préférence entre 10 µm et 35 µm, mesuré selon l'annexe C de la norme EN 868-3 ; et/ou
il a un indice COBB à 60 secondes, mesuré selon la norme ISO 535, inférieur à 20 g/m² ; et/ou
il respecte la norme DIN 58953-6 sections 3 et 4 en termes de barrière bactérienne ; et/ou
il respecte la norme ISO 11607-1 ; et/ou
il a une épaisseur d'au moins 200, de préférence de 300 µm ; et/ou
il est calandré ; et/ou
le matériau a une résistance à la flexion d'au moins 1000 mN dans un sens machine et un sens travers, de préférence d'au moins 2000 mN, et encore plus préférablement d'au moins 3000 mN, selon la norme ISO 2493-2, Papier et carton - Détermination de la résistance à la flexion, Partie 2 : Rigidimètre Taber ; et/ou
le matériau a un taux global de transmission de vapeur d'eau (WVTR) d'au moins 400 g/m²/jour, de préférence d'au moins 800 g/m²/jour, et plus préférablement d'au moins 1000 g/m²/jour à 38°C et 90 % d'humidité relative et tel que déterminé par la norme ISO 2528.

9. Matériau selon l'une quelconque des revendications précédentes, **caractérisé en ce qu'**il comprend en outre un agent de résistance à l'état humide, représentant entre 0,15 et 1 % en poids sec par rapport au poids sec de cellulose.

10. Matériau selon la revendication 9, **caractérisé en ce que** l'agent de résistance à l'état humide est choisi dans le groupe comprenant les résines polyamine-épichlorhydrine (PAE) glyoxalées, telles la résine glyoxalées polyamide (GPAM) à base de formaldéhyde.

11. Matériau selon l'une quelconque des revendications précédentes, **caractérisé en ce qu'**il comprend en outre un agent d'encollage représentant entre 0,15 et 1 % en poids sec par rapport au poids sec de cellulose.

12. Matériau selon la revendication 11, **caractérisé en ce que** l'agent d'encollage est choisi dans le groupe comprenant le dimère d'alkylcétène (AKD), l'anhydride alcénylsuccinique (ASA) et une résine à base de colophane.

13. Matériau selon l'une quelconque des revendications précédentes, **caractérisé en ce qu'**il comprend en outre une couche de revêtement dont la composition est apte à la rendre thermoscellable.

14. Matériau selon l'une quelconque des revendications précédentes, **caractérisé en ce que** le mélange de fibres comprend en outre entre 1 et 40 % en poids de fibres de cellulose mercerisées.

15. Plateau obtenu à partir du matériau spécifié par l'une quelconque des revendications 1 à 14.

16. Utilisation du matériau spécifié par l'une quelconque des revendications 1 à 14 pour la fabrication d'un plateau.

17. Emballage pour stérilisation médicale, comprenant :
un plateau selon la revendication 15 ; et
un moyen de fermeture étanche stérilisable du plateau.

18. Récipient pour l'emballage de dispositifs médicaux destinés à être stérilisés comprenant :
un plateau comprenant un matériau fibreux stérilisable, le plateau s'étendant d'une première extrémité à une seconde extrémité, et s'étendant d'un premier côté à un second côté, la première extrémité incluant une première paroi d'extrémité et la seconde extrémité incluant une seconde paroi d'extrémité, et le premier côté incluant une première paroi latérale et le second côté incluant une seconde paroi latérale, les première et seconde parois d'extrémité et les première et seconde parois latérales définissant un évidement ou une cavité à l'intérieur des limites internes des première et seconde parois d'extrémité et des première et seconde parois latérales, et avec une paroi de fond au fond de l'évidement ou la cavité ;
dans lequel le plateau a un poids de base d'au moins 180 g/m², avantageusement d'au moins 200 g/m² ; et
dans lequel le plateau comprend un mélange de fibres contenant au moins 75 % en poids de fibres de cellulose naturelle, dont la longueur est inférieure à 5 mm ; et
dans lequel le plateau a une perméabilité à l'air d'au moins 1,7, de préférence d'au moins 3,4, avantageusement strictement supérieure à 3,4 µm/Pa.s à une pression de 1,47 kPa, mesurée selon la norme ISO 5636-3 ; et
dans lequel le plateau a des pores ayant un diamètre de pore maximal inférieur à 50 µm, mesuré selon l'annexe C de la norme EN 868-3.

19. Procédé de fabrication d'un récipient pour l'emballage de dispositifs médicaux destinés à être stérilisés comprenant :
la fourniture d'un matériau fibreux stérilisable ; et
le formage du matériau fibreux stérilisable en un plateau, le plateau s'étendant d'une première extrémité à une seconde extrémité, et s'étendant d'un premier côté à un second côté, la première extrémité incluant une première paroi d'extrémité et la seconde extrémité incluant une seconde paroi d'extrémité, et le premier côté incluant une première paroi latérale et le second côté incluant une seconde paroi latérale, les première et seconde parois d'extrémité et les première et seconde parois latérales définissant un évidement ou une cavité à l'intérieur des limites internes des première et seconde parois d'extrémité et des première et seconde parois latérales, et avec une paroi de fond au fond de l'évidement ou la cavité ;
dans lequel le plateau a un poids de base d'au moins 180 g/m², avantageusement d'au moins 200 g/m² ; et
dans lequel le plateau comprend un mélange de fibres contenant au moins 75 % en poids de fibres de cellulose naturelle, dont la longueur est inférieure à 5 mm ; et
dans lequel le plateau a une perméabilité à l'air d'au moins 1,7, de préférence d'au moins 3,4, avantageusement strictement supérieure à 3,4 µm/Pa.s à une pression de 1,47 kPa, mesurée selon la norme ISO 5636-3 ; et
dans lequel le plateau a des pores ayant un diamètre de pore maximal inférieur à 50 µm, mesuré selon l'annexe C de la norme EN 868-3.

20. Procédé selon la revendication 19, dans lequel le formage du matériau fibreux stérilisable en plateau comprend le thermoformage du matériau fibreux stérilisable ou le moulage du matériau fibreux stérilisable.
